# EUROPÄISCHE PATENTSCHRIFT

(11) **EP 1 050 275 B1**
(45) Veröffentlichungstag und Bekanntmachung des Hinweises auf die Patenterteilung: **21.09.2005**
(21) Anmeldenummer: 00107946.6
(22) Anmeldetag: 14.04.2000
(51) Int. Cl.: A61B 17/32

(54) **Medizinisches Instrument zum Präparieren von Gewebe**
Medical instrument for preparing tissue
Instrument médical pour préparer des tissus

(30) Priorität: 29.04.1999 EP 99108380
(43) Veröffentlichungstag der Anmeldung: 08.11.2000
(60) Teilanmeldung: 05012958.4
(73) Patentinhaber: Karl Storz GmbH & Co. KG, 78532 Tuttlingen (DE)
(72) Erfinder: Lang, Dieter, 96342 Stockheim (DE); Bacher, Uwe, 78532 Tuttlingen (DE); Anders, Fridolin, 78194 Immendingen (DE)
(74) Vertreter: Heuckeroth, Volker

(56) Entgegenhaltungen:
- DE-A- 4 300 064
- DE-A- 19 520 717
- DE-U- 8 712 835
- US-A- 4 646 745
- US-A- 5 507 772

## Beschreibung

Die Erfindung betrifft ein medizinisches Instrument zum Präparieren von Gewebe im menschlichen oder tierischen Körper nach dem Oberbegriff des Anspruchs 1.

Ein solches medizinisches Instrument ist aus der DE 43 00 064 A1 bekannt.

Bei einem Instrument zum Präparieren von Gewebe handelt es sich um ein Instrument zum Abtrennen und/oder Fassen von Gewebe, beispielsweise um eine Stanze, Schere oder Zange.

Derartige Instrumente werden im Rahmen der minimal-invasiven Chirurgie dazu verwendet, Gewebe im menschlichen oder tierischen Körper, üblicherweise unter endoskopischer Sichtkontrolle, abzutrennen. Dazu weisen derartige Instrumente einen lang erstreckten Schaft auf, an dessen distalem Ende zumindest ein bewegliches Werkzeug angeordnet ist, das üblicherweise mit einem weiteren beweglichen oder unbeweglichen Werkzeug am distalen Ende des Schafts zum Abtrennen des Gewebes zusammenwirkt. Zum Betätigen des zumindest einen beweglichen Werkzeuges weisen derartige Instrumente ferner am proximalen Ende des Schafts zumindest ein bewegliches Griffteil auf, das über ein auf Schub und/oder Zug arbeitendes axial bewegliches Kraftübertragungselement kraftschlüssig mit dem zumindest einen beweglichen Werkzeug verbunden ist, so daß eine Bewegung des beweglichen Griffteils vom Kraftübertragungselement in eine Bewegung des beweglichen Werkzeugs übertragen wird.

Insbesondere bei operativen Eingriffen im Hals-Nasen-Ohrenbereich werden Instrumente verwendet, deren Schaft zumindest eine Biegung aufweist, um das oder die Werkzeuge am distalen Ende an schwer zugängliche Stellen, beispielsweise in Nischen der Stirn- oder Kieferhöhle, bringen zu können.

Bei einem Instrument mit einem gebogenen Schaft, insbesondere, wenn der Biegeradius klein ist, stellt sich das Problem, die Kraft vom beweglichen Griffteil über das Kraftübertragungselement durch die Biegung hindurch auf das bewegliche Werkzeug zu übertragen. Dies ist durch den Wunsch nach schlanken, d.h. dünnschaftigen Instrumenten besonders erschwert.

Um sich an die Biegung anpassen zu können, muß das Kraftübertragungselement daher im Bereich der Biegung des Schafts eine Biegeelastizität aufweisen.

Aus der DE 44 44 025 A1 ist beispielsweise ein Kraftübertragungselement für ein medizinisches Instrument in Form eines biegeelastischen Drahtelements offenbart, das sich an eine derartige Biegung anpassen kann. Ein solcher flexibler Draht kann jedoch nur Zugkräfte übertragen, ist jedoch nicht geeignet, Schubkräfte, insbesondere hohe Schubkräfte, zu übertragen. Wird das Kraftübertragungselement nämlich auf Schub belastet, knickt der Draht aus, so daß nur eine geringe oder keine Schubkraft auf das zumindest eine bewegliche Werkzeug übertragen werden kann, um dieses zum Abtrennen von Gewebe zu betätigen.

Da mit derartigen Instrumenten unter anderem auch hartes Gewebe, beispielsweise Knorpel- oder Knochengewebe, abgetrennt werden soll, muß das Kraftübertragungselement daher so beschaffen sein, daß auch sehr hohe Kräfte, insbesondere hohe Schubkräfte, auf das bewegliche Werkzeug übertragen werden können, ohne daß das Kraftübertragungselement dabei ausknickt, wobei die Kraftübertragung andererseits wohlgemerkt über die Biegung des Schafts erfolgen muß, das Kraftübertragungselement sich bei seiner axialen Bewegung also dem Biegungsverlauf anpassen können muß.

Die DE 195 20 717 C2 offenbart zur Lösung dieses Problems ein Rohrschaftinstrument mit einem rohrförmigen Schaft, der eine Biegung aufweist, wobei das Kraftübertragungselement im Innern des Rohrschafts angeordnet ist. Das Kraftübertragungselement ist im gebogenen Bereich des Rohrschafts als Stab aus einem biegeelastischen Material ausgebildet, der an der Innenwand einer ihn umgebenden starren, konzentrisch zum Schaft verlaufenden Hülse anliegt, und dessen Querschnitt im Bereich der Biegung durch eine Anzahl von axial nebeneinander angeordneten Umfangsnuten bereichsweise reduziert ist. Die zuvor genannte Hülse kann dabei auch durch den Rohrschaft selbst gebildet sein. Die Umfangsnuten sind an dem Stab voll umfänglich ausgebildet. Im Bereich der Umfangsnuten ist der Stab somit drahtförmig verdünnt mit rundem Querschnitt ausgebildet. In den Zwischenbereichen zwischen den Nuten ist der Durchmesser des Stabs nicht verringert, so daß die Außenflächen der Zwischenbereiche als Führungsflächen dienen, um das Kraftübertragungselement im Bereich der Biegung an der Innenwand der Hülse bzw. des Rohrschaftes zu führen.

Mit einem solchen Kraftübertragungselement lassen sich zwar hohe Schubkräfte durch die Biegung hindurch auf das bewegliche Werkzeug übertragen, dennoch kann dieses bekannte Instrument mit dieser Ausgestaltung des Kraftübertragungselements als nachteilig angesehen werden.

Da derartige Instrumente im Rahmen der minimal-invasiven Chirurgie verwendet werden, besteht nämlich eine weitere Anforderung an derartige Instrumente darin, daß ihr Durchmesser im Bereich des Schaftes möglichst klein ist, um das Instrument mit dem Schaft durch eine möglichst kleine Inzision oder natürliche Körperöffnung in den Körper einführen zu können. Bei derartigen miniaturisierten Instrumenten mit einem Schaftdurchmesser von wenigen Millimetern bedeutet dies für das bekannte Instrument, daß das Kraftübertragungselement in den verdünnten Bereichen der Umfangsnuten einen sehr kleinen Durchmesser aufweist, wodurch die Stabilität des Kraftübertragungselements verringert ist, da sich die Materialstärke des Kraftübertragungselements im Bereich der Umfangsnuten auf sehr dünne Drahtabschnitte reduziert. Im Bereich der Umfangsnuten kann es daher bei der Übertragung hoher Schubkräfte oder bei stoßartigen Schubkräften zu einem Knicken, Brechen oder bei Übertragung hoher Zugkräfte zu einem Reißen des Kraftübertragungselements im Bereich der vollumfänglich ausgebildeten Umfangsnuten kommen, wodurch die Betriebssicherheit des bekannten Instruments verringert ist.

Die bereits genannte DE 43 00 064 A1 offenbart eine Gewebestanze mit einem Außenschaft und einem Innenschaft, an dessen distalem Ende eine Öffnung mit einer Schneide vorgesehen ist, die mit einer Gegenschneide am distalen Ende des Außenschaftes so zusammenarbeitet, daß bei Betätigung der Gewebestanze Gewebe, das durch die erwähnte Öffnung in den Innenschaft ragt, durch die gegeneinander bewegten Schneiden abgetrennt wird. Der Innenschaft ist starr ausgebildet, verläuft im proximalen Bereich gerade und geht distalwärts in einen gekrümmten Verlauf über, und der Außenschaft, der das Kraftübertragungselement dieses Instruments bildet, ist zumindest im Bereich dieser Krümmung verformbar. Die Verformbarkeit des Außenschaftes wird dadurch ermöglicht, daß dieser mit Ausnehmungen versehen ist, die sich auf den Radien der Krümmung des Innenschaftes gegenüberliegen. Die Ausnehmungen in Form von radialen Einschnitten belassen lediglich eine schmale Materialbrücke zwischen sich.

Die US 5,507,772 offenbart ebenfalls ein medizinisches Instrument, dessen Schaft eine Biegung aufweist. Das Kraftübertragungselement weist eine Mehrzahl von eingekerbten oder ausgesparten Abschnitten im Bereich der Biegung des Schafts auf. Zwischen den ausgesparten Abschnitten des Kraftübertragungselements sind eine Mehrzahl von Rippen ausgebildet, deren Dicke im wesentlichen der Dicke des übrigen Körpers des Kraftübertragungselements entspricht. Die Rippen sind nur zu einer Seite des Kraftübertragungselements hin ausgebildet, während das Kraftübertragungselement auf der den Rippen gegenüberliegenden Seite im Bereich der Biegung durchgehend flach ausgebildet ist. Das Kraftübertragungselement und dessen Rippen sind in einem länglichen Schlitz innerhalb des Schafts geführt, der in der konkaven Innenseite der Biegung des Schafts vorhanden ist. In einem anderen Ausführungsbeispiel sind die Rippen weggelassen, so daß das Kraftübertragungselement in diesem Fall im Bereich der Biegung als flaches Band ausgebildet ist, das dann aber nicht geführt ist.

Aus der US 4,646,745 ist weiterhin ein medizinisches Klammerinstrument bekannt, das ein in einem Schaft angeordnetes Kraftübertragungselement aufweist, wobei das Kraftübertragungselement in Form eines Flachbandes ausgebildet ist, so daß das Kraftübertragungselement flexibel ist. Das Flachband selbst besteht aus drei Lagen von dünnen Bändern. Im Bereich einer Biegung des Schafts ist das Flachband entlang eines Abstandselements geführt, um zu erreichen, daß das Flachband im Bereich der Biegung seine zentrische Lage im Schaft beibehält, wenn es unter Zugspannung steht, d.h. sich nicht in der Biegung des Schafts gerade zieht. Das Abstandselement ist mit Kugellagern zur Verminderung der Reibung am Kraftübertragungselement versehen.

Der Erfindung liegt die Aufgabe zugrunde, ein medizinisches Instrument der eingangs genannten Arten dahingehend weiterzubilden, daß mittels des Kraftübertragungselements über die Biegung des Schaftes hinweg hohe Zug- und/oder Schubkräfte auf das zumindest eine bewegliche Werkzeug übertragen werden können, ohne daß das Kraftübertragungselement dabei ausknickt, und ohne daß im Bereich der Biegung eine Verringerung der Stabilität des Kraftübertragungselements auftritt.

Erfindungsgemäß wird diese Aufgabe hinsichtlich des eingangs genannten medizinischen Instruments dadurch gelöst, daß das rohrförmige Element als Schraubenfeder mit eng aneinanderliegenden Windungen ausgebildet ist.

Bei dem erfindungsgemäßen Instrument ist demnach vorgesehen, das Kraftübertragungselement zumindest im Bereich der Biegung als der Biegung entsprechendes biegeelastisches rohrförmiges Element in Form einer Schraubenfeder auszubilden, die am Schaft anliegend geführt ist. Ein solches rohrförmiges Element hat den Vorteil, daß es durchgehend mit im wesentlichen gleicher Materialstärke ausgebildet werden kann. Dadurch, daß das rohrförmige Element im Bereich der Biegung am Schaft anliegend geführt ist, wird ein Ausknicken des rohrförmigen Elements vermieden, weil ein seitliches Ausweichen des rohrförmigen Elements beim Übertragen von Schubkräften durch die Führung am Schaft nicht möglich ist. Die Ausgestaltung des-Kraftübertragungselements des erfindungsgemäßen Instruments eignet sich sowohl für den Fall, daß das Kraftübertragungselement in einem als Rohrschaft ausgebildeten Schaft innenliegend angeordnet ist, als auch für den Fall, daß das Kraftübertragungselement selbst als Rohrschaft ausgebildet ist und außen am Schaft geführt ist. Dadurch, daß das rohrförmige Element axial durchgehend mit gleicher Materialstärke ausgebildet werden kann, werden Verdünnungsbereiche wie bei dem bekannten Instrument, bei dem sich das Kraftübertragungselement in den Bereichen der Umfangsnuten drahtförmig verdünnt, vermieden, wodurch eine insgesamt höhere Stabilität und somit höhere Betriebssicherheit des Kraftübertragungselements und damit des gesamten Instruments erreicht wird.

Die Ausgestaltung des rohrförmigen Elements in Form der Schraubenfeder ist besonders vorteilhaft, weil eine Schraubenfeder, die beispielsweise aus Stahl ausgebildet ist, in radialer Richtung starr ist, jedoch bezüglich ihrer Längsachse eine hohe Biegeelastizität aufweist, weil sich die Windungen der Schraubenfeder beim Biegen der Schraubenfeder relativ zueinander bewegen können. Dadurch, daß die Schraubenfeder eng aneinanderliegende Windungen aufweist, können ohne Kraftverlust hohe Schubkräfte durch die Schraubenfeder und ohne Verzögerung auf das bewegliche Werkzeug übertragen werden, weil die Schraubenfeder axial nicht komprimierbar ist.

In einer bevorzugten Ausgestaltung ist das Kraftübertragungselement als Rohr ausgebildet, das im Bereich der Biegung das rohrförmige Element aufweist, wobei das Rohr um den Schaft herum angeordnet ist.

Diese Maßnahme, bei der das Kraftübertragungselement bezüglich des Schafts außenliegend ist, hat den besonderen Vorteil, insbesondere bei miniaturisierten Instrumenten mit geringem Schaftdurchmesser, daß der Schaft selbst relativ schmal bauend ausgebildet werden kann, während für das Kraftübertragungselement ein größerer Durchmesser zur Verfügung steht. Dadurch kann auch bei besonders schmal bauenden Instrumenten ein besonders stabiles und zur Übertragung hoher Schub- und Zugkräfte geeignetes Kraftübertragungselement geschaffen werden.

In einer weiteren bevorzugten Ausgestaltung weist der Schaft einen massiven Stab aus einem Vollmaterial auf.

Hierbei ist von Vorteil, daß durch den als massiven Stab ausgebildeten Schaft ein besonders stabiler Instrumentenkörper gebildet wird, der dem Instrument eine besonders hohe Stabilität gegen Biegebeanspruchungen des Schafts verleiht.

Weiterhin ist es bevorzugt, wenn das rohrförmige Element mit dem übrigen Rohr stoffschlüssig verbunden ist.

Diese Maßnahme ist hinsichtlich der einfachen Herstellbarkeit des Kraftübertragungselements von Vorteil, da das rohrförmige Element, insbesondere in Form einer Schraubenfeder, und das übrige Rohr des Kraftübertragungselements in getrennten Fertigungsgängen hergestellt und anschließend durch Löten, Schweißen oder Kleben miteinander verbunden werden können. Ein weiterer Vorteil dieser Maßnahme besteht darin, daß die Schraubenfeder und das übrige Rohr nur ein Bauteil darstellen, wodurch bei einem in Schaft und Kraftübertragungselement zerlegbaren Instrument die Montage und Demontage des Instruments vereinfacht wird.

In einer weiteren bevorzugten Ausgestaltung ist das rohrförmige Element direkt mit dem beweglichen Werkzeug verbunden.

Hierbei ist von Vorteil, daß im Fall, daß sich die Biegung nahe am distalen Ende des Schafts befindet, eine axial kurz bauende Bauweise des Instruments distalseitig der Biegung ermöglicht wird.

In einer weiteren bevorzugten Ausgestaltung ist das bewegliche Werkzeug mittels eines flexiblen Zugelements parallel zum rohrförmigen Element mit dem übrigen Rohr kraftschlüssig verbunden.

Hierbei ist von Vorteil, daß auch hohe Zugkräfte über die Biegung hinweg übertragen werden können, wobei die Zugkräfte dann von dem flexiblen Zugelement aufgenommen werden, wodurch außerdem vermieden wird, daß sich bei Zugbeanspruchung des Kraftübertragungselements das rohrförmige Element, wenn dieses eine Schraubenfeder aufweist, axial dehnt, wodurch stets gewährleistet ist, daß das bewegliche Werkzeug auch bei Zugkraftübertragung mitbewegt wird. Das Zugelement kann dabei in Form eines Bandes, Drahtes oder einer Litze aus Metall, Kohlefaser oder Kevlar gebildet sein.

Dabei ist es weiterhin bevorzugt, wenn der Schaft im Bereich der Biegung eine Nut aufweist, in der das Zugelement zwischen dem rohrförmigen Element und dem Schaft geführt ist.

Hierbei ist von Vorteil, daß auch das Zugelement bei den axialen Bewegungen des Kraftübertragungselements über die Biegung hinweg zwischen der Schraubenfeder und dem Schaft mit wenig Spiel geführt und gehalten wird.

In einer weiteren bevorzugten Ausgestaltung ist das Zugelement direkt mit dem zumindest einen beweglichen Werkzeug verbunden.

Durch diese Maßnahme wird vorteilhafterweise gewährleistet, daß das zumindest eine bewegliche Werkzeug bei Auftreten einer Zugkraft auf das flexible Zugelement stets bewegt wird.

In einer weiteren bevorzugten Ausgestaltung des erfindungsgemäßen Instruments sind der Schaft und das Kraftübertragungselement voneinander abnehmbar.

Hierbei ist von Vorteil, daß sich Verunreinigungen, die sich im Bereich des rohrförmigen Elements und auch im übrigen Bereich des Schafts zwischen dem Kraftübertragungselement und dem Schaft ansammeln, leicht und sicher entfernt werden können, wodurch die Reinigbarkeit des erfindungsgemäßen Instruments verbessert ist.

In einer weiteren bevorzugten Ausgestaltung ist das zumindest eine bewegliche Werkzeug relativ zum Schaft axial beweglich, wobei ein zweites Werkzeug am distalen Ende des Schafts angeordnet ist, wobei das bewegliche Werkzeug und das zweite Werkzeug durch axiale Relativbewegung zueinander in Form einer Stanze zusammenwirken.

Eine solche Ausgestaltung hat insbesondere in sehr beengten Operationsgebieten, beispielsweise in der Stirn- oder Kieferhöhle, den Vorteil, daß die beiden Werkzeuge zum Abtrennen von Gewebe keinen über den Umfang des Schafts hinausgehenden freien Arbeitsradius benötigen, weil beide Werkzeuge lediglich axial in Längsrichtung des Schafts, genauer gesagt in Längsrichtung des distalen Endes des Schafts, relativ zueinander bewegt werden.

Dabei ist es weiterhin bevorzugt, wenn das zweite Werkzeug unbeweglich mit dem Schaft verbunden ist und distal vor dem beweglichen Werkzeug angeordnet ist.

Hierbei ist von Vorteil, daß das zweite unbewegliche Werkzeug die distale Spitze des Schafts bildet und beim Ansetzen dieses unbeweglichen Werkzeugs an dem abzutrennenden Gewebe einen ortsfesten Arbeitspunkt definiert. In diesem Fall arbeitet das Kraftübertragungselement auf Schub, um das bewegliche Werkzeug nach distal gegen das unbewegliche Werkzeug zu schieben, um zwischen dem unbeweglichen Werkzeug und dem beweglichen Werkzeug befindliches Gewebe abzutrennen, und auf Zug, um beide Werkzeuge wieder voneinander zu trennen.

In einer weiteren bevorzugten Ausgestaltung ist das zumindest eine bewegliche Werkzeug gegenüber dem zweiten Werkzeug verdrehgesichert.

Diese Maßnahme ist insbesondere im Falle des medizinischen Instruments von Vorteil, bei dem das Kraftübertragungselement als Rohr und im Bereich der Biegung als Schraubenfeder ausgebildet ist. Die Verdrehsicherung hält das bewegliche Werkzeug und das zweite Werkzeug in Drehrichtung um die Längsachse des Schafts lagefest zueinander, auch wenn auf die Schraubenfeder Torsionskräfte wirken sollten.

Alternativ zu der Ausgestaltung des zumindest einen beweglichen Werkzeuges als Stanzwerkzeug läßt sich die Erfindung jedoch vorteilhaft einsetzen, wenn das zumindest eine bewegliche Werkzeug als um ein Drehgelenk verschwenkbares Maulteil ausgebildet ist, das mit einem zweiten Werkzeug in der Art einer Schere oder Zange zusammenwirkt.

Weitere Vorteile ergeben sich aus der nachfolgenden Beschreibung und der beigefügten Zeichnung.

Ausführungsbeispiele der Erfindung sind in der Zeichnung dargestellt und werden hiernach mit Bezug auf diese näher beschrieben. Es zeigen:
- Fig. 1: ein Ausführungsbeispiel eines erfindungsgemäßen medizinischen Instruments in einer Gesamtdarstellung in einer teilweise geschnittenen Seitenansicht;
- Fig. 2: eine Darstellung der Anordnung aus Schaft und Kraftübertragungselement des Instruments in Fig. 1 in teilweise geschnittener Seitenansicht in vergrößertem Maßstab;
- Fig. 3: den Schaft im Bereich seines distalen Endes in Alleinstellung, ebenfalls in vergrößertem Maßstab;
- Fig. 4: einen Schnitt entlang der Linie IV-IV in Fig. 2;
- Fig. 5: einen Schnitt entlang der Linie V-V in Fig. 3;
- Fig. 6: einen Schnitt entlang der Linie VI-VI in Fig. 3;
- Fig. 7: ein unbewegliches Griffteil des Instruments in Fig. 1 in Alleinstellung in teilweise geschnittener Seitenansicht;
- Fig. 8: ein Teil des beweglichen Griffteils in Alleinstellung in Rückansicht, über das ein proximales Ende des Kraftübertragungselements mit dem beweglichen Griffteil verbunden ist;
- Fig. 9: einen Schnitt entlang der Linie IX-IX in Fig. 8; und
- Fig. 10: einen Schnitt entlang der Linie X-X in Fig. 1.

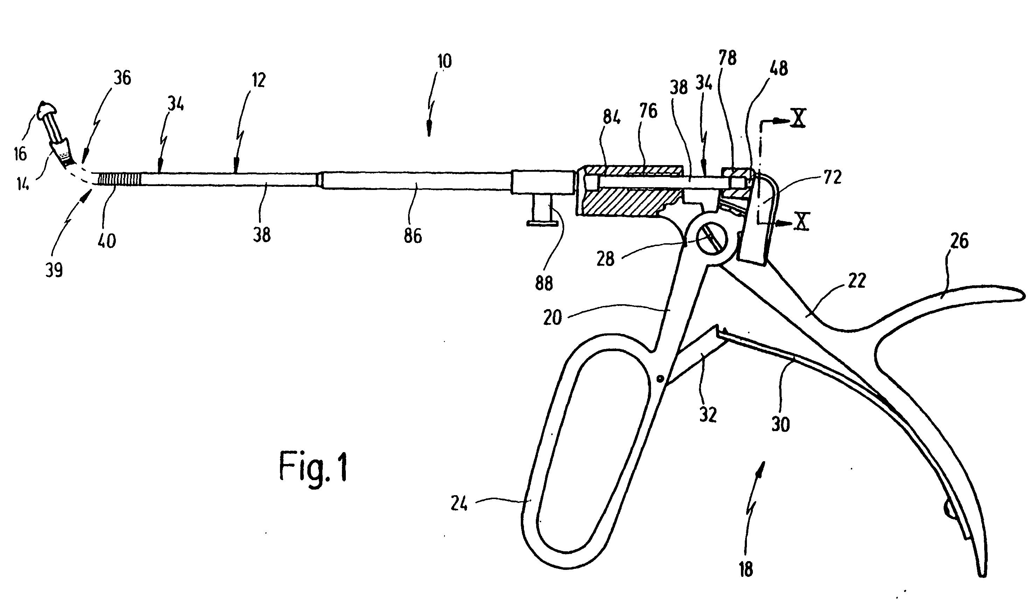

In Fig. 1 ist ein mit dem allgemeinen Bezugszeichen 10 versehenes medizinisches Instrument dargestellt. Das Instrument 10 dient zum Abtrennen von Gewebe im menschlichen oder tierischen Körper.

Das Instrument 10 weist einen lang erstreckten Schaft 12 auf. An einem distalen Ende des Schafts 12 ist ein bewegliches Werkzeug 14 angeordnet. Weiterhin ist am distalen Ende des Schafts 12 distal vor dem beweglichen Werkzeug 14 ein unbewegliches Werkzeug 16 angeordnet.

Am proximalen Ende des Schafts 12 ist eine Handhabe 18 angeordnet, die ein bewegliches Griffteil 20 und ein unbewegliches Griffteil 22 aufweist.

Das bewegliche Griffteil 20 weist einen Ring 24 zum Durchstecken eines, zweier oder dreier Finger auf. Das unbewegliche Griffteil 22 weist einen nach proximal abstehenden Fortsatz 26 auf, an dem sich bei der Handhabung des Instruments 10 die Mulde zwischen Daumen und Zeigefinger abstützt.

Das bewegliche Griffteil 20 und das unbewegliche Griffteil 22 sind über ein Drehgelenk 28 gelenkig miteinander verbunden, wobei eine Drehachse des Drehgelenks 28 senkrecht zur Längsrichtung des Schafts 12 verläuft.

Weiterhin ist an dem unbeweglichen Griffteil 22 eine Blattfeder 30 befestigt, die mit einem Hebel 32 verbunden ist, der an dem beweglichen Griffteil 20 gelenkig befestigt ist. Die Blattfeder 30 und der Hebel 32 spannen das bewegliche Griffteil 20 in seine in Fig. 1 dargestellte, von dem unbeweglichen Griffteil 22 beabstandete Offenstellung vor, in der die Werkzeuge 14 und 16 ebenfalls voneinander beabstandet sind.

Das bewegliche Griffteil 20 ist mit dem beweglichen Werkzeug 14 über ein axial bewegliches Kraftübertragungselement 34 kraftschlüssig verbunden, das hiernach noch näher beschrieben wird.

Weiterhin weist der Schaft 12 eine Biegung 36 auf, die sich im Bereich des distalen Endes des Schafts 12 befindet. Eine Krümmungsebene der Biegung 36 liegt dabei in Fig. 1 in der Zeichenebene.

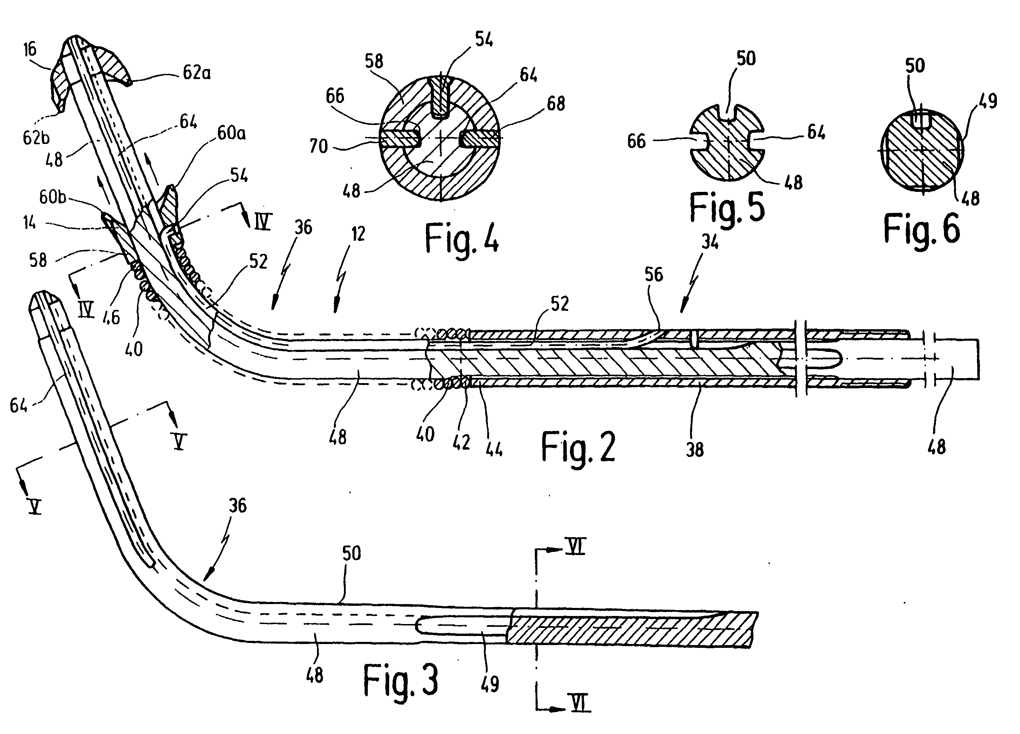

Gemäß Fig. 1 und 2 ist das Kraftübertragungselement 34 als Rohr 38 ausgebildet. Im Bereich der Biegung 36 ist das Kraftübertragungselement 34 als der Biegung entsprechend biegeelastisches rohrförmiges Element 39 in Form einer Schraubenfeder 40 ausgebildet, die sich über die Biegung 36 hinweg erstreckt. Die Schraubenfeder 40 weist eine Vielzahl von eng aneinander liegenden Windungen aus einem Federstahl auf, wobei in Fig. 1 und 2 die Schraubenfeder 40 nur im Bereich ihres distalen und im Bereich ihres proximalen Endes dargestellt ist. Es versteht sich jedoch, daß sich die Schraubenfeder 40 mit ihren eng anliegenden Windungen über den gesamten Bereich der Biegung 36 hinweg erstreckt.

Ein proximales Ende 42 der Schraubenfeder 40 ist mit einem distalen Ende 44 des Rohrs 38 stoffschlüssig, beispielsweise durch Laserschweißen, verbunden. Ein distales Ende 46 der Schraubenfeder 40 ist mit dem beweglichen Werkzeug 14 an dessen proximalem Ende stoffschlüssig, beispielsweise ebenfalls durch Laserschweißen, verbunden, wodurch eine kraftschlüssige Verbindung zwischen dem Kraftübertragungselement 34 und dem beweglichen Werkzeug 14 hergestellt wird.

Der Schaft 12 weist weiterhin einen Stab 48 auf. Der Stab 48 ist aus einem Vollmaterial massiv und starr ausgebildet. Das Rohr 38 und die damit verbundene Schraubenfeder 40 sind um den Stab 48 herum angeordnet und entlang demselben bei ihrer axialen Bewegung umfänglich geführt. Dabei entspricht der Innendurchmesser der Schraubenfeder 40 und des Rohrs 38 dem Außendurchmesser des Stabs 48.

Der Stab 48 weist im Querschnitt gemäß Fig. 5 und 6 einen runden Querschnitt auf, wobei der Schaft im Bereich proximal hinter der Biegung 36 vier umfänglich begrenzte Flachseiten 49 aufweist, so daß im Bereich der Flachseiten 49 zwischen dem Rohr 38 und dem Stab 48 Zwischenräume verbleiben, die als Spülquerschnitte genutzt werden können, um zwischen dem Rohr 38 und dem Stab 48 eine Spülflüssigkeit in das Operationsgebiet zu leiten.

Der Stab 48 weist im Bereich der Biegung 36 auf seiner dem Innenradius der Biegung 36 zugewandten Seite eine Nut 50 auf, in der ein flexibles Zugelement 52 aufgenommen ist. Das flexible Zugelement 52 ist in Form eines biegeelastischen Drahtes ausgebildet. Ein distales Ende 54 des Zugelements 52 ist stoffschlüssig, beispielsweise durch Schweißen, mit dem beweglichen Werkzeug 14 verbunden. Vom beweglichen Werkzeug 14 aus liegt das flexible Zugelement 52 in der Nut 50 des Stabes 48 innerhalb der Schraubenfeder 40 und ist mit einem proximalen Ende 56 stoffschlüssig mit dem Rohr 38 in einer in diesem vorgesehenen Bohrung ebenfalls stoffschlüssig verbunden. Das flexible Zugelement 52 verhindert beim Zurückziehen des Rohrs 38 eine Dehnung der Schraubenfeder 40, wenn also das Kraftübertragungselement 34 auf Zug arbeitet.

Das bewegliche Werkzeug 14 ist axial beweglich auf dem Stab 48 angeordnet und weist dazu an seinem proximalen Ende einen Hülsenabschnitt 58 auf, der den Stab 48 umfänglich umgibt. Der Hülsenabschnitt 58 weist insgesamt eine nur geringe axiale Ausdehnung auf.

Am äußersten distalen Ende des Stabs 48 ist das unbewegliche Werkzeug 16 fest mit dem Stab 48 verbunden, wobei die feste Verbindung zwischen dem unbeweglichen Werkzeug 16 und dem Stab 48 ebenfalls stoffschlüssig durch Schweißen hergestellt ist. In Fig. 3 ist das Werkzeug 16 weggelassen.

Das bewegliche Werkzeug 14 und das unbewegliche Werkzeug 16 sind als Stanzwerkzeuge ausgebildet und weisen dazu geschärfte Bereich 60a und 60b bzw. 62a und 62b auf, die in Form von Schneidzähnen ausgebildet sind. Die geschärften Bereiche 60a, 60b sowie 62a, 62b wirken zum Abtrennen von Gewebe schneidend zusammen, wenn das bewegliche Werkzeug 14 aus der in Fig. 1 und 2 dargestellten Position nach distal gegen das unbewegliche Werkzeug 16 geschoben bzw. gedrückt wird.

Im Bereich der Werkzeuge 14 und 16 weist der Stab 48 weiterhin seitliche Nuten 64 und 66 auf, in die fest mit dem Hülsenabschnitt 58 des beweglichen Werkzeugs 14 verbundene Stege 68 und 70 eingreifen. Durch das System aus den Nuten 64 und 66 und den Stegen 68 und 70 wird eine Verdrehsicherung für das bewegliche Werkzeug 14 an dem Stab 48 geschaffen, so daß das unbewegliche Werkzeug 16 und das bewegliche Werkzeug 14 stets in Drehrichtung um die Längsachse des Schafts lagefest zueinander gehalten werden.

Wieder mit Bezug auf Fig. 1 erstrecken sich das Rohr 38 und der Stab 48 gemeinsam bis zur Handhabe 18 am proximalen Ende des Schafts 12.

Der Stab 48 ist dabei bis zu einer Halterung 72 geführt, die fest und unbeweglich mit dem unbeweglichen Griffteil 22 verbunden ist.

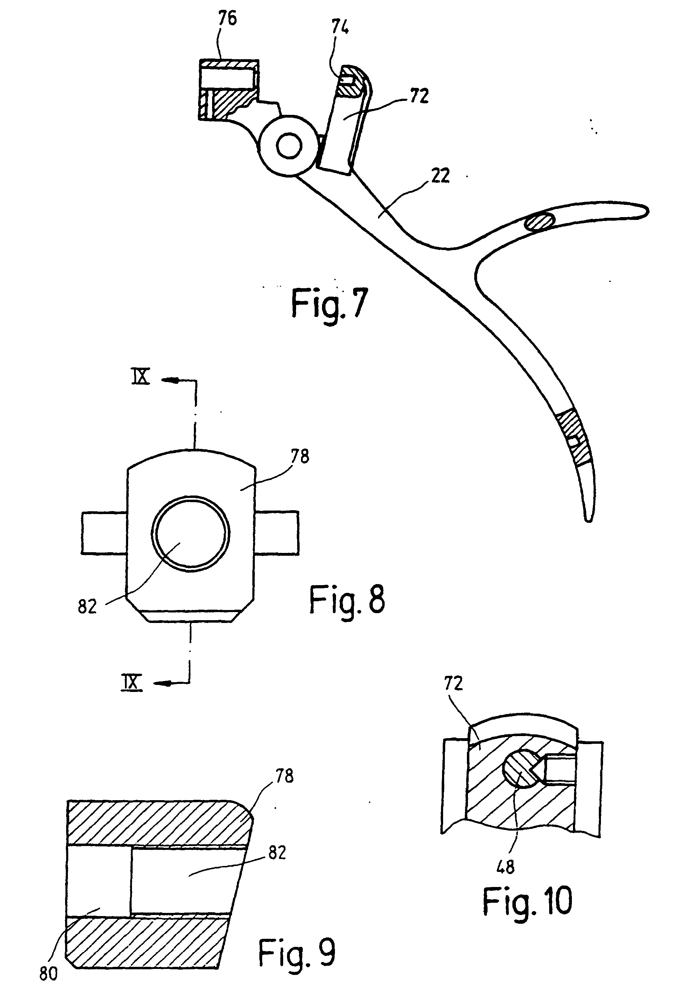

In Fig. 7 ist das unbewegliche Griffteil 22 in Alleinstellung dargestellt. In der Halterung 72 ist eine Bohrung 74 vorhanden, in der das äußerste proximale Ende des Stabs 48 aufgenommen und beispielsweise mit einer seitlich in die Halterung 72 eingedrehten Schraube (nicht dargestellt) fest verankert ist (vgl. auch Fig. 10).

Das unbewegliche Griffteil 22 weist ferner ein feststehendes Rohrstück 76 auf, durch das das Rohr 38 und der Stab 48 durchgeführt sind. Während der Stab 48 relativ zu dem Rohrstück 76 unbeweglich ist, ist das Rohr 38 relativ zu dem Rohrstück 76 axial verschiebbar.

Das Rohr 38 ist seinerseits kraftschlüssig mit einem Reiter 78 verbunden, der an dem beweglichen Griffteil 20 befestigt ist. Der Reiter 78 ist in Fig. 8 und 9 in Alleinstellung dargestellt. Während das äußerste proximale Ende des Rohrs 38 in einem distalen Bereich 80 einer Bohrung 82 mit dem Reiter 78 fest verbunden ist, ist der Stab 48 durch die Bohrung 82 bis zu der Halterung 72 durchgeführt. Der Reiter 78 ist demnach relativ zu dem Stab 48 axial beweglich.

Gemäß Fig. 1 schließt sich distal an das Rohrstück 76 weiterhin ein fest mit dem unbeweglichen Griffteil 22 verbundenes Rohrstück 84 an, in dem ein Führungsrohr 86 befestigt ist, das somit unbeweglich ist. Durch das Führungsrohr 86 hindurch erstreckt sich das Rohr 38 des Kraftübertragungselements 34 sowie der Stab 48 des Schaftes 12.

In dem Rohrstück 84 ist ferner ein Anschluß 88 zum Anschließen einer Spülleitung angeordnet, so daß die Spülflüssigkeit zwischen das Rohr 38 und den Stab 48 eingeleitet und zum distalen Ende des Schafts 12 hin geleitet werden kann, wie bereits zuvor erwähnt.

Bei dem Instrument 10 ist weiterhin der Stab 48 aus dem Rohr 38 herausnehmbar, was trotz der Biegung 36 durch die Schraubenfeder 40 im Bereich der Biegung 36 ermöglicht wird. Um das Herausziehen des Stabs 48 aus dem Kraftübertragungselement 34 in Form des Rohres 38 und der Schraubenfeder 40 und dem damit verbundenen beweglichen Werkzeug 14 zu ermöglichen, ist das bewegliche Werkzeug 14, insbesondere dessen Hülsenabschnitt 58, axial kurz gebaut, so daß der Stab 48 mit seiner Biegung 36 durch das bewegliche Werkzeug 14 herausgezogen werden kann. Zusätzlich erstrecken sich dann die Nuten 50, 64 und 66 entgegen der Darstellung in Fig. 2 und 3 über die gesamte Länge des Stabs 48.

Im folgenden wird nun die Funktionsweise des Instruments 10 beschrieben.

Ausgehend von der in Fig. 1 dargestellten Betriebsstellung, in der das bewegliche Werkzeug 14 in seiner maximal proximalen Stellung ist, wird durch Bewegen des beweglichen Griffteils 20 in Richtung auf das unbewegliche Griffteil 22 hin der Reiter 78 und damit das damit fest verbundene Rohr 38 axial nach distal geschoben, während der Stab 48 dabei feststeht. Von dem Rohr 38 wird die axiale Bewegung über die Schraubenfeder 40 über die Biegung 36 des Schafts 12 hinweg auf das bewegliche Werkzeug 14 übertragen, wodurch dieses auf das unbewegliche Werkzeug 16 hin verschoben wird. Das Kraftübertragungselement 34 arbeitet somit zum Abtrennen von Gewebe auf Schub. Aufgrund der eng aneinander liegenden Windungen der Schraubenfeder 40 wird diese durch die Schubkraft des Rohrs 38 nicht gestaucht, sondern die Kraft wird unmittelbar auf das bewegliche Werkzeug 14 übertragen. Durch Zurückbewegen des beweglichen Griffteils 20 in die in Fig. 1 dargestellte Stellung bewirkt nun das flexible Zugelement 52 in Fig. 2, daß das bewegliche Werkzeug 14, wenn das Kraftübertragungselement 34 auf Zug arbeitet, sicher wieder zurückgezogen wird, da das flexible Zugelement 52 verhindert, daß sich die Schraubenfeder 40 beim Zurückziehen des Rohrs 38 axial dehnt.

## Patentansprüche

1. Medizinisches Instrument zum Präparieren von Gewebe im menschlichen oder tierischen Körper, mit einem Schaft (12), mit zumindest einem beweglichen Werkzeug (14) an einem distalen Ende des Schafts (12), mit zumindest einem beweglichen Griffteil (20) an einem proximalen Ende des Schafts (12), und mit einem sich längs des Schafts (12) erstreckenden relativ zu diesem beweglichen, auf Schub und/oder Zug arbeitenden Kraftübertragungselement (34), dessen proximales Ende mit dem zumindest einen beweglichen Griffteil (20) und dessen distales Ende mit dem zumindest einen beweglichen Werkzeug (14) kraftschlüssig verbunden ist, wobei der Schaft (12) an zumindest einer Stelle zwischen dem distalen Ende und dem proximalen Ende eine Biegung (36) aufweist, wobei das Kraftübertragungselement (34) zumindest im Bereich der Biegung (36) als der Biegung entsprechend biegeelastisches rohrförmiges Element (39) ausgebildet ist, das am Schaft (12) anliegend geführt ist, **dadurch gekennzeichnet, daß** das rohrförmige Element (39) als Schraubenfeder (40) mit eng aneinanderliegenden Windungen ausgebildet ist.

2. Instrument nach Anspruch 1, **dadurch gekennzeichnet, daß** das Kraftübertragungselement (34) als Rohr (38) ausgebildet ist, das im Bereich der Biegung das rohrförmige Element (39) aufweist, wobei das Rohr (38) um den Schaft (12) herum angeordnet ist.

3. Instrument nach Anspruch 2, **dadurch gekennzeichnet, daß** der Schaft (12) einen massiven Stab (48) aus einem Vollmaterial aufweist.

4. Instrument nach Anspruch 2 oder 3, **dadurch gekennzeichnet, daß** das rohrförmige Element (39) mit dem übrigen Rohr (38) stoffschlüssig verbunden ist.

5. Instrument nach einem der Ansprüche 1 bis 4, **dadurch gekennzeichnet, daß** das rohrförmige Element (39) direkt mit dem beweglichen Werkzeug (14) verbunden ist.

6. Instrument nach einem der Ansprüche 1 bis 5, **dadurch gekennzeichnet, daß** das bewegliche Werkzeug (14) mittels eines flexiblen Zugelements (52) parallel zum rohrförmigen Element (39) mit dem übrigen Rohr (38) kraftschlüssig verbunden ist.

7. Instrument nach Anspruch 6, **dadurch gekennzeichnet, daß** der Schaft (12) im Bereich der Biegung (36) eine Nut (50) aufweist, in der das Zugelement (52) zwischen dem rohrförmigen Element (39) und dem Schaft (12) geführt ist.

8. Instrument nach Anspruch 6 oder 7, **dadurch gekennzeichnet, daß** das Zugelement (52) direkt mit dem zumindest einen beweglichen Werkzeug (14) verbunden ist.

9. Instrument nach einem der Ansprüche 1 bis 8, **dadurch gekennzeichnet, daß** der Schaft (12) und das Kraftübertragungselement (34) voneinander abnehmbar sind.

10. Instrument nach einem der Ansprüche 1 bis 9, **dadurch gekennzeichnet, daß** das zumindest eine bewegliche Werkzeug (14) relativ zum Schaft (12) axial beweglich ist, wobei ein zweites Werkzeug (16) am distalen Ende des Schafts (12) angeordnet ist, wobei das bewegliche Werkzeug (14) und das zweite Werkzeug (16) durch axiale Relativbewegung zueinander in Form einer Stanze zusammenwirken.

11. Instrument nach Anspruch 10, **dadurch gekennzeichnet, daß** das zweite Werkzeug (16) unbeweglich mit dem Schaft (112) verbunden ist und distal vor dem beweglichen Werkzeug (14) angeordnet ist.

12. Instrument nach Anspruch 10 oder 11, **dadurch gekennzeichnet, daß** das bewegliche Werkzeug (14) relativ zum zweiten Werkzeug (16) verdrehgesichert ist.

13. Instrument nach einem der Ansprüche 1 bis 9, **dadurch gekennzeichnet, daß** das zumindest eine bewegliche Werkzeug als um ein Drehgelenk verschwenkbares Maulteil ausgebildet ist, das mit einem zweiten Werkzeug in der Art einer Schere oder Zange zusammenwirkt.

## Claims

1. Medical instrument for dissecting tissue in the human or animal body, comprising a shaft (12), at least one movable tool (14) at a distal end of the shaft (12), at least one movable grip element (20) at a proximal end of the shaft (12), and a force transmission element (34) that extends along the shaft (12) and is movable relative to the latter and is operable for transmitting push and/or pull forces, and whose proximal end is connected to the at least one movable grip element (20) in force-locking fashion and whose distal end is connected to the at least one movable tool (14) in force-locking fashion, the shaft having a bend (36) at at least one location between the distal end and the proximal end, wherein the force transmission element (34) is configured, at least in the area of the bend (36), as a flexible tubular element (39) according to the bend, which is guided in contact with the shaft (12), **characterized in that** the tubular element (39) is configured as a helical spring (40) with closely wound windings.

2. The instrument of claim 1, **characterized in that** the force transmission element (34) is configured as a tube (38) comprising the tubular element (39) in the area of a bend, the tube (38) being arranged around the shaft (12).

3. The instrument of claim 2, **characterized in that** the shaft (12) comprises a solid bar (48) made from a solid material.

4. The instrument of claim 2 or 3, **characterized in that** the tubular element (39) is connected with the remaining tube (38) by a substance bond.

5. The instrument of anyone of claims 1 through 4, **characterized in that** the tubular instrument (39) is directly connected with the movable tool (14).

6. The instrument of anyone of claims 1 through 5, **characterized in that** the movable tool (14) is connected with the remaining tube (38) in force-locking fashion, by means of a flexible tension element (52) parallel to the tubular element (39).

7. The instrument of claim 6, **characterized in that** the shaft (12) comprises a groove (50), in the area of the bend, in which the tension element (52) is guided between the tubular element (39) and the shaft (12).

8. The instrument of claim 6 or 7, **characterized in that** the tension element (52) is directly connected with the at least one movable tool (14).

9. The instrument of anyone of claims 1 through 8, **characterized in that** the shaft (12) and the force transmission element (34) are detachable one from the other.

10. The instrument of anyone of claims 1 through 9, **characterized in that** the at least one movable tool (14) is axially movable relative to the shaft (12) and a second tool (16) is arranged at the distal end of the shaft (12), the movable tool (14) and the second tool (16) acting together in the way of a punch if moved axially one relative to the other.

11. The instrument of claim 10, **characterized in that** the second tool (16) is connected with the shaft immovably, and is arranged distally before the movable tool (14).

12. The instrument of claim 10 or 11, **characterized in that** the movable tool (14) is protected from torsion relative to the second tool (16).

13. The instrument of anyone of claims 1 through 9, **characterized in that** the at least one movable tool (1) is configured as jaw part which can be pivoted about a hinge and which coacts with a second tool in the manner of a pair of scissors or a forceps.

## Revendications

1. Instrument médical pour la préparation de tissu dans l'organisme humain ou animal, avec une tige (12), avec au moins un outil mobile (14) à une extrémité distale de la tige (12), avec au moins une partie de poignée mobile (20) à une extrémité proximale de la tige (12), et avec un élément de transmission de force (34) s'étendant le long de la tige (12), mobile par rapport à celle-ci et travaillant en poussée et/ou en traction, dont l'extrémité proximale est reliée selon une liaison positive à ladite au moins une partie de poignée mobile (20) et l'extrémité distale audit au moins un outil mobile (14), dans lequel la tige (12) présente à un endroit au moins entre l'extrémité distale et l'extrémité proximale une courbure (36), l'élément de transmission de force (34) étant réalisé au moins dans la zone de la courbure (36) sous la forme d'un élément tubulaire (39) flexible élastiquement qui est guidé en contact sur la tige (12), **caractérisé en ce que** l'élément tubulaire (39) est réalisé sous la forme d'un ressort hélicoïdal (40) avec des spires en contact étroit les unes avec les autres.

2. Instrument selon la revendication 1, **caractérisé en ce que** l'élément de transmission de force (34) est réalisé sous la forme d'un tube (38) qui présente l'élément tubulaire (39) dans la zone de la courbure, le tube (38) étant disposé autour de la tige (12).

3. Instrument selon la revendication 2, **caractérisé en ce que** la tige (12) présente une baguette massive (48) en matériau plein.

4. Instrument selon la revendication 2 ou la revendication 3, **caractérisé en ce que** l'élément tubulaire (39) est relié par friction au reste du tube (38).

5. Instrument selon l'une des revendications 1 à 4, **caractérisé en ce que** l'élément tubulaire (39) est relié directement à l'outil mobile (14).

6. Instrument selon l'une des revendications 1 à 5, **caractérisé en ce que** l'outil mobile (14) est relié selon une liaison positive au moyen d'un élément de traction flexible (52), parallèlement à l'élément tubulaire (39), au reste du tube (38).

7. Instrument selon la revendication 6, **caractérisé en ce que** la tige (12) présente dans la zone de la courbure (36) une rainure (50) dans laquelle l'élément de traction (52) est guidé entre l'élément tubulaire (39) et la tige (12).

8. Instrument selon la revendication 6 ou la revendication 7, **caractérisé en ce que** l'élément de traction (52) est relié directement audit au moins un outil mobile (14).

9. Instrument selon l'une des revendications 1 à 8, **caractérisé en ce que** la tige (12) et l'élément de transmission de force (34) peuvent être désolidarisés.

10. Instrument selon l'une des revendications 1 à 9, **caractérisé en ce que** ledit au moins un outil mobile (14) est mobile axialement par rapport à la tige (12), un deuxième outil (16) étant disposé à l'extrémité distale de la tige (12), l'outil mobile (14) et le deuxième outil (16) coopérant par un mouvement axial relatif l'un par rapport à l'autre à la manière d'une poinçonneuse.

11. Instrument selon la revendication 10, **caractérisé en ce que** le deuxième outil (16) est relié de manière immobile à la tige (112) et disposé distalement avant l'outil mobile (14).

12. Instrument selon la revendication 10 ou 11, **caractérisé en ce que** l'outil mobile (14) est verrouillé en torsion par rapport au deuxième outil (16).

13. Instrument selon l'une des revendications 1 à 9, **caractérisé en ce que** ledit au moins un outil mobile est réalisé sous la forme d'une partie de mâchoire, pouvant pivoter autour d'une articulation tournante, qui coopère avec un deuxième outil à la manière d'une cisaille ou d'une pince.
